# EUROPÄISCHE PATENTSCHRIFT

(11) **EP 4 577 635 B1**
(45) Veröffentlichungstag und Bekanntmachung des Hinweises auf die Patenterteilung: **22.04.2026**
(21) Anmeldenummer: 23755079.3
(22) Anmeldetag: 10.08.2023
(51) Int. Cl.: C12M 1/22, C12M 3/00, C12M 1/12

(54) **AUFNAHMEGERÄT FÜR EINEN NÄHRMEDIUMTRÄGER**
RECEIVING DEVICE FOR A NUTRIENT MEDIUM CARRIER
APPAREIL DE RÉCEPTION POUR UN SUBSTRAT DE MILIEU DE CULTURE

(30) Priorität: 22.08.2022 EP 22191545
(43) Veröffentlichungstag der Anmeldung: 02.07.2025
(73) Patentinhaber: Pharmabotix AG, 5707 Seengen (CH)
(72) Erfinder: GLANZMANN, Luca, 6212 St. Erhard (CH)
(74) Vertreter: Rutz, Andrea
(86) Internationale Anmeldenummer: PCT/EP2023/072218
(87) Internationale Veröffentlichungsnummer: WO 2024/041909

(56) Entgegenhaltungen:
- EP-A2- 1 460 126
- WO-A1-2014/086984
- WO-A1-2020/084324
- WO-A1-2020/233854
- WO-A1-2021/156263
- WO-A1-2022/132014
- WO-A1-97/08337
- CN-A- 102 174 395
- CN-A- 112 080 423

## Beschreibung

### TECHNISCHES GEBIET

Die vorliegende Erfindung betrifft ein Aufnahmegerät für einen Nährmediumträger, wie insbesondere eine Petrischale. Das Aufnahmegerät ist zum Einbringen in einen hermetisch verschliessbaren Isolator ausgebildet, um Keime in der Atmosphäre des Isolators zu erkennen. Die Erfindung betrifft daher auch ein Verfahren zum Erkennen von Keimen in der Atmosphäre eines Isolators.

### STAND DER TECHNIK

Aseptische Isolatoren kommen insbesondere in der Pharmazie zum Einsatz, um gegenüber dem umgebenden Arbeitsraum eine definierte, keimfreie Atmosphäre zu erzeugen, damit empfindliche oder gefährliche Produkte verarbeitet werden können. Isolatoren werden beispielsweise oft als Teil von Füllanlagen verwendet, welche zum Befüllen von Behältnissen, wie zum Beispiel Vials, Ampullen oder Spritzen, mit einem biopharmazeutischen Produkt dienen. Aufgrund der hochreinen und insbesondere keimfreien Atmosphäre im Isolator kann während des Abfüllvorgangs das Risiko einer Kontamination des Produkts durch Keime, wie zum Beispiel Viren oder Bakterien, erheblich reduziert oder bestenfalls sogar weitgehend ausgeschlossen werden. Die Behältnisse werden noch im Isolator hermetisch verschlossen, bevor sie dem Isolator zwecks Lagerung, Transport und Vertrieb entnommen werden. Die Sterilität der im verschlossenen Behältnis aufgenommenen Produkte kann dadurch zu einem hohen Grad gewährleistet werden.

Die Atmosphäre in derartigen Isolatoren wird üblicherweise im Rahmen eines sog. Bio-Monitorings überwacht, um eine mikrobiologische Kontamination des Isolatorraums auch während einer länger andauernden Verwendung des Isolators stets sicherstellen zu können. Hierzu werden im Isolatorraum z.B. Petrischalen positioniert, die ein geeignetes Nährmedium tragen. Das Nährmedium wird dadurch der Atmosphäre im Isolatorraum ausgesetzt. Allfällige in der Isolatoratmosphäre vorhandene Mikroorganismen setzen sich in der Petrischale ab und wachsen dort zu detektierbaren Kolonien heran.

Die Handhabung der Petrischalen, insbesondere die Positionierung sowie das Öffnen und Wiederverschliessen mit einem Deckel, im Inneren des Isolators erfolgt im Stand der Technik üblicherweise mittels eines Handschuheingriffs. Bei den flexibel ausgebildeten Handschuhdurchführungen besteht das Risiko einer Beschädigung und eines dadurch entstehenden Lecks. Keime, die sich im Nährboden abgesetzt und allenfalls dort bereits vermehrt haben, können zudem bei der Handhabung mittels des Handschuheingriffs verschleppt und dadurch im Isolator verbreitet werden.

Es ist deshalb bekannt, im Inneren des Isolators einen Roboter mit Greifarmen vorzusehen, welcher die Handhabung der Nährmediumträger, d.h. der Petrischalen, übernimmt, so dass auf den Handschuheingriff verzichtet werden kann.

Beispielsweise ist in der WO 2020/233854 A1 eine Verpackungsanlage zum Abfüllen und Verschliessen von Medikamente in Behältnisse offenbart, welche einen 6-Achs-Roboter zur Handhabung der Petrischalen aufweist.

Die WO 2021/156263 A1 offenbart ein Verfahren zum automatisierten Keim-Monitoring in einem Isolator. Der Transfer der Nährbodenträger von einer Transferschleuse zu einem jeweils vorgesehenen Nährbodenträgerhalter erfolgt dabei robotergestützt.

Roboter, insbesondere Mehrgelenkroboter und Roboter mit Greifarmen, sind jedoch in der Regel komplex im Aufbau und dadurch teuer. Die Steuerung bzw. Programmierung von Robotern ist zudem meist schwierig und nur durch ausgebildete Fachpersonen möglich. Eine Anpassung von einfachen Parametern, wie zum Beispiel eine andere Positionierung der Petrischalen, ist deshalb oft nicht ohne weiteres möglich. Des Weiteren ist die Nachrüstung von bestehenden Isolatoren mit einem Roboter aufwändig bzw. oft gar nicht möglich.

EP 1 460 126 offenbart eine Abfüllanlage in dessen sterilisierten Arbeitskammer ein Keim-Monitoring stattfindet. Eine mittels Roboterarm mit Ansaugkopf gesteuerte Handhabungsvorrichtung sorgt dafür, dass der Deckel einer Petrischale abgehoben wird zum Beginn des Keim-Monitorings und ausgetauscht wird sowie bei bevorstehender H₂O₂-Dampf-Dekontamination des Arbeitsraums wieder verschlossen wird.

### DARSTELLUNG DER ERFINDUNG

Es ist deshalb eine Aufgabe der Erfindung, ein Gerät anzugeben, das eine einfache und sichere Handhabung von Nährmediumträgern in einem Isolator erlaubt. Das Gerät sollte eine möglichst einfache Konstruktion aufweisen und zudem wenn möglich ohne viel Aufwand auch bei bestehenden Isolatoren zum Einsatz kommen können.

Zur Lösung dieser Aufgabe wird ein Aufnahmegerät für einen Nährmediumträger, wie insbesondere eine Petrischale, vorgeschlagen, wie es in Anspruch 1 angegeben ist. Ausserdem wird in Anspruch 11 ein Verfahren zur Erkennung von Keimen in der Atmosphäre eines Isolators angegeben. Vorteilhafte Ausgestaltungen der Erfindung sind in den abhängigen Ansprüchen angegeben.

Die vorliegende Erfindung stellt also ein Aufnahmegerät für einen Nährmediumträger, wie insbesondere eine Petrischale, zur Verfügung, aufweisend
einen hermetisch verschliessbaren Innenraum zur Aufnahme des Nährmediumträgers; sowie
einen drahtlos fernbedienbaren Antrieb zum Öffnen und Verschliessen des Aufnahmegeräts, um den Zugang zum Nährmediumträger freizugeben bzw. den Innenraum mit dem darin aufgenommenen Nährmediumträger hermetisch zu verschliessen.

Das Aufnahmegerät kann als Ganzes in einen hermetisch isolierbaren Raum eines Isolators eingebracht und diesem entnommen werden.

Indem das Aufnahmegerät einen drahtlos fernbedienbaren Antrieb zum Öffnen und Verschliessen aufweist und mit dem darin aufgenommenen Nährmediumträger als Ganzes in den Isolator eingebracht werden kann, ist keine weitere Handhabung des Nährmediumträgers im Inneren des hermetisch isolierten Isolatorraums z.B. mittels eines Handschuheingriffs oder mittels Roboterarmen notwendig. Die Handhabung kann dadurch ausschliesslich mittels einer drahtlosen Fernbedienung auf eine sichere sehr einfache Art und Weise erfolgen. Hierzu wird der Nährmediumträger ausserhalb des Isolators unter einer Schutzatmosphäre in das Aufnahmegerät eingesetzt, welches dann hermetisch verschlossen wird. Das Aufnahmegerät kann dann in den Isolatorraum eingebracht werden, wo es im immer noch verschlossenen Zustand von aussen sterilisiert werden kann, ohne dass das Wachstums- bzw. Nährmedium während dem Dekontaminieren Schaden nimmt. Wenn der Isolator für die Produktion bereit ist, kann der Antrieb des Aufnahmegeräts mittels der Fernbedienung aktiviert werden, um dieses zu öffnen und den Zugang zum Nährmediumträger freizugeben. Das Nährmedium wird dadurch der Atmosphäre des Isolatorraums ausgesetzt. Nach einer gewissen Einwirkzeit kann das Aufnahmegerät mittels der Fernbedienung wieder verschlossen werden, so dass keine Keime vom Nährmediumträger nach aussen und in die umgekehrte Richtung gelangen können. In diesem Zustand kann das Aufnahmegerät zusammen mit dem darin aufgenommenen, von der Umgebung hermetische isolierte Nährmediumträger aus dem Isolator herausgenommen werden. Der Nährmediumträger kann dann dem Aufnahmegerät entnommen und zur Bebrütung und/oder Mikroorganismendetektion weiter transportiert werden. Alternativ kann der Weitertransport auch mitsamt dem Aufnahmegerät durchgeführt werden.

Dadurch, dass das Aufnahmegerät mitsamt dem Nährmediumträger als Ganzes in den Isolator einbringbar und diesem entnehmbar ist, eignet es sich zudem besonders gut zur Verwendung in bestehenden Isolatoren bzw. zur Nachrüstung. Der Anwendungsbereich liegt insbesondere bei sogenannten "Gloveless"-Isolatoren, also Isolatoren, die keine Eingriffshandschuhe aufweisen. Im Vergleich zum einem typischen Roboterarm ist das Aufnahmegerät ausserdem nicht nur einfacher bedien- und steuerbar, sondern typischerweise auch erheblich einfacher konstruierbar und dadurch in der Regel insgesamt kostengünstiger.

Beim Nährmediumträger kann es sich insbesondere um eine Petrischale handeln. Das Nährmedium ist dann im Inneren der Petrischale aufgenommen, wobei die Petrischale insbesondere einen Deckel aufweisen kann. Bevorzugt weist der Nährmediumträger eine Schale und einen Deckel auf, wobei der Deckel vorteilhaft mit der Schale verriegelbar ist. Dadurch ist der Nährmediumträger ausserhalb des Aufnahmegeräts sicher und insbesondere derart transportierbar, dass das darin enthaltene Nährmedium von der Umgebung geschützt ist. Es ist aber auch durchaus möglich, dass der Nährmediumträger eine Schale und einen Deckel aufweist, welche nicht miteinander verriegelbar sind. Grundsätzlich ist es aber auch denkbar, dass es sich beim Nährmediumträger um eine einfache Platte handelt, welche das Nährmedium trägt.

Als eine Petrischale wird eine flache, runde, meist durchsichtige Schale angesehen, welche einen Deckel aufweisen kann, aber nicht muss. Falls die Petrischale einen Deckel aufweist, kann dieser die Schale über- oder untergreifen, das heisst der Deckelrand kann sich in radialer Richtung über die Schale hinauserstrecken oder komplett innerhalb der Schale angeordnet sein.

Der Nährmediumträger, insbesondere die Petrischale, kann ein Teil des Aufnahmegeräts bilden und z.B. zusammen mit diesem vertrieben werden.

Bei verschlossenem Aufnahmegerät ist der Innenraum des Aufnahmegeräts somit hermetisch von der Umgebung isoliert. Allfällig vorhandene Keime können dann somit weder in den Innenraum hinein noch von diesem nach aussen gelangen. Falls ein Nährmediumträger im Innenraum des verschlossenen Aufnahmegeräts aufgenommen ist, ist somit auch dieses hermetisch von der Umgebung isoliert.

Der Antrieb dient zum Öffnen und Schliessen des Aufnahmegeräts, d.h. zum Freigeben bzw. hermetischen Isolieren des Innenraums. Beim Antrieb handelt es sich bevorzugt um einen Motor, insbesondere einen Elektromotor. Die Fernbedienung des Antriebs kann beispielsweise über Infrarot, Funk, Wi-Fi bzw. WLAN oder Bluetooth erfolgen. Das Aufnahmegerät weist hierzu bevorzugt eine entsprechend ausgebildete und je nachdem eigens dafür vorgesehene Fernbedienung auf. Bei der Fernbedienung kann es sich aber um ein Smartphone handeln, auf welcher eine App zur Bedienung des Aufnahmegeräts installiert ist.

Unter einem "Isolator" wird allgemein ein Behälter verstanden, dessen Innenraum gegenüber einem umgebenden Arbeitsraum hermetisch isolierbar, d.h. abtrennbar, ist. Im Innenraum kann eine definierte Atmosphäre zur Bearbeitung empfindlicher oder gefährlicher Produkte, wie insbesondere pharmazeutischer Produkte, erzeugt werden. Beim Isolator kann es sich um einen stationären oder um einen mobilen Isolator handeln. Der Isolator steht üblicherweise in einem Raum eines Gebäudes, welcher einen den Isolator umgebenden Arbeitsraum bildet. Dies im Gegensatz zu einem Reinraum von z.B. einem Labor- oder einem anderen Gebäude, welcher selbst den Arbeitsraum bildet. Der Isolator kann insbesondere einen Teil einer Füllanlage bilden, welche z.B. zum Befüllen von Behältnissen mit einem pharmazeutischen Produkt dient.

Das Aufnahmegerät weist bevorzugt ausserdem zumindest ein Energiespeicherelement, wie insbesondere eine Batterie, zur Versorgung des Antriebs mit elektrischer Energie auf. Bei der Batterie kann es sich um eine aufladbare oder um eine Einweg-Batterie handeln. Das Vorhandensein eines Energiespeicherelements macht die Handhabung des Aufnahmegeräts beim Einbringen in den Isolator bzw. bei der Entnahme aus dem Isolator besonders einfach.

In einer insbesondere bevorzugten Ausführungsform ist das Aufnahmegerät dazu ausgebildet, beim Öffnen einen Deckel des Nährmediumträgers von einer Schale des Nährmediumträgers abzuheben. Der Nährmediumträger kann dadurch ausserhalb des Isolators verschlossen in das Aufnahmegerät eingesetzt und erst im hermetisch isolierten Raum des Isolators geöffnet werden. Bevorzugt ist das Aufnahmegerät zudem dazu ausgebildet, beim Verschliessen den Deckel wieder auf die Schale aufzusetzen. Der Nährmediumträger kann dadurch noch im Isolator wieder verschlossenen werden.

Bevorzugt weist das Aufnahmegerät ein erstes Halteelement auf zum Festhalten des Deckels des Nährmediumträgers. Das erste Halteelement kann insbesondere zum Öffnen und/oder Verschliessen des Nährmediumträgers beim Öffnen bzw. Schliessen des Aufnahmegeräts dienen. Ausserdem weist das Aufnahmegerät bevorzugt ein zweites Halteelement zum Festhalten der Schale des Nährmediumträgers auf. Das zweite Halteelement kann insbesondere zum Öffnen und/oder Verschliessen des Nährmediumträgers beim Öffnen bzw. Schliessen des Aufnahmegeräts dienen. Wenn sowohl das erste Halteelement als auch das zweite Halteelement vorhanden sind, kann der Nährmediumträger besonders sicher geöffnet und wieder verschlossen werden.

Beim ersten Halteelement handelt es sich insbesondere bevorzugt um eines oder mehrere Klemmelemente, welche den Deckel des Nährmediumträgers vorzugsweise von einer oder mehreren radialen Richtungen her festklemmen. Auch wenn dies nicht bevorzugt ist, könnte das erste Halteelement alternativ auch durch ein Fixierelement gebildet sein, welches von unten her am Deckel anliegt, das heisst in diejenige Richtung am Deckel anliegt, in welche dieser beim Öffnen von der Schale abgehoben wird.

Das zweite Haltelement wird bevorzugt durch ein Fixierelement gebildet, welches von oben her auf der Schale aufliegt, das heisst entgegen der Richtung auf der Schale aufliegt, in welche der Verschlussdeckel beim Öffnen von der Schale abgehoben wird. Das Fixierelement, welches insbesondere als ein Fixierring ausgebildet sein kann, liegt dabei bevorzugt auf dem äusseren radialen Rand der Schale auf. Bevorzugt klemmt das Fixierelement die Schale dabei zudem fest, so dass sie bei verschlossenem Innenraum des Aufnahmegeräts gegenüber dem Fixierelement nicht gedreht werden kann. Eine solche Festklemmung der Schale kann insbesondere dazu dienen, eine Drehung des Verschlussdeckels gegenüber der Schale zu ermöglichen, um den Nährmediumträger mittels eines Bajonett- oder Gewindeverschlusses zu entriegeln oder zu verriegeln. Grundsätzlich ist es aber auch denkbar, dass es sich beim zweiten Halteelement wie bei der oben erwähnten bevorzugten Variante des ersten Halteelements um eines oder mehrere Klemmelemente handelt, welche die Schale des Nährmediumträgers vorzugsweise von einer oder mehreren radialen Richtungen her festklemmen.

Der Nährmediumträger weist wie erwähnt bevorzugt eine Schale auf, welche von einem Deckel verschliessbar ist. Von der Schale und dem Deckel wird dann bevorzugt gemeinsam ein Innenraum begrenzt, der zur Aufnahme eines Nährmediums dient. Der Deckel ist bevorzugt mit der Schale verriegelbar. Dadurch wird ein sicherer Transport des Nährmediumträgers ausserhalb des Aufnahmegeräts ermöglicht. In einer insbesondere bevorzugten Ausführungsform ist das Aufnahmegerät dazu ausgebildet, den im hermetisch verschlossenen Innenraum des Aufnahmegeräts aufgenommenen Nährmediumträger zu entriegeln und zu verriegeln, das heisst, den Deckel gegenüber der Schale zu entriegeln bzw. mit dieser zu verriegeln, um den Innenraum des Nährmediumträgers zugänglich zu machen bzw. zu verschliessen. Die Handhabung des Nährmediumträgers wird dadurch besonders sicher, da dieser dann im Inneren des hermetisch verschlossenen Isolators entriegelt und geöffnet sowie wieder verschlossen und verriegelt werden kann.

Zum Entriegeln und Verriegeln des Nährmediumträgers im Innenraum des Aufnahmegeräts weist das Aufnahmegeräts bevorzugt ein Betätigungselement auf, welches eine Entriegelung und Verriegelung des Nährmediumträgers von Hand erlaubt. Das Aufnahmegerät ist dadurch besonders einfach herstellbar und bedienbar. Das Betätigungselement bildet bevorzugt ein Teil des weiter unten angegebenen Verriegelungsmechanismus'. In anderen Ausführungsformen kann es aber auch vorteilhaft sein, wenn das Aufnahmegerät ein Betätigungselement aufweist, das motorisch angetrieben ist, d.h. eine automatische Entriegelung und Verriegelung des Nährmediumträgers erlaubt.

Der Nährmediumträger kann insbesondere einen Gewinde- oder Bajonettverschluss aufweisen. Ein Bajonettverschluss ist ein Verschluss, bei dem die beiden zu verbindenden Teile durch Ineinanderstecken und anschliessendes entgegengesetztes Drehen miteinander verbunden werden. Gemäss einer insbesondere bevorzugten Ausführungsform weist das Aufnahmegerät einen Verriegelungsmechanismus auf zur Entriegelung und Verriegelung eines solchen Nährmediumträgers mit einem Gewinde- oder Bajonettverschluss. Der Verriegelungsmechanismus ist hierzu bevorzugt dazu ausgebildet, den Deckel gegenüber der Schale des Nährmediumträgers zu drehen und, im Falle eines Bajonettverschlusses, den Deckel und die Schale vorgängig in- oder aufeinander zu stecken. Um eine sichere Funktion des Verriegelungsmechanismus' zu gewährleisten, weist dieser hierzu bevorzugt eine oder mehrere Kulissenführungen auf. Die eine oder mehreren Kulissenführen dienen vorteilhaft dazu, eine Führung bereitzustellen für das für das Auf- oder Ineinanderstecken von Deckel und Schale und/oder für das Drehen des Deckels gegenüber der Schale. Da viele kommerziell erhältliche Nährmediumträger und insbesondere Petrischalen einen Gewinde- oder Bajonettverschluss aufweisen, ist eine solche Ausführungsform des Aufnahmegeräts besonders vorteilhaft. Ein Gewinde- oder Bajonettverschluss erlaubt zudem eine besonders einfache und dennoch sichere Verriegelung eines Deckels an einer Schale eines Nährmediumträgers.

Der Verriegelungsmechanismus weist bevorzugt ein erstes Halteelement auf zum Festhalten des Deckels eines Nährmediumträgers, wobei das erste Halteelement aus einer Ausgangsstellung in eine niedergedrückte Stellung und aus der niedergedrückten Stellung in eine niedergedrückte gedrehte Stellung bewegbar ist, um einen Nährmediumträger, welcher einen Bajonettverschluss aufweist, zu verriegeln. Falls das Aufnahmegerät ein zweites Halteelement aufweist, dient dieses dann bevorzugt dazu, die Schale des Nährmediumträgers während des Niederdrückens und Drehen des ersten Halteelements in einer unbeweglichen Position festzuhalten.

Der Verriegelungsmechanismus ist bevorzugt dazu ausgebildet, zur Entriegelung und Verriegelung von einem Benutzer von Hand bedient zu werden. Grundsätzlich ist es aber auch denkbar, dass ein Motor vorhanden ist, um den Verriegelungsmechanismus zu bedienen. Der Motor kann, muss aber nicht, durch den bereits erwähnten Antrieb gebildet sein.

Das Aufnahmegerät weist bevorzugt ein Aufnahmegefäss und einen Verschlussdeckel auf, welche gemeinsam den zur Aufnahme des Nährmediumträgers vorgesehenen Innenraum begrenzen. Bevorzugt wird der Innenraum im geschlossenen Zustand des Aufnahmegeräts ausschliesslich durch das Aufnahmegefäss und den Verschlussdeckel begrenzt. Das Aufnahmegefäss und/oder der Verschlussdeckel weist bevorzugt ein Dichtungselement auf, um den Innenraum hermetisch gegenüber der Umgebung abzudichten. Das Dichtungselement erstreckt sich vorteilhaft vollständig umlaufend um den Innenraum herum. Bevorzugt ist der Verschlussdeckel zum Öffnen und Verschliessen des Innenraums über einen Winkelbereich von mindestens 45°, bevorzugter von mindestens 90°, noch bevorzugter von sogar mindestens 180°, um ein Drehgelenk schwenkbar. Eine Verschwenkung des Verschlussdeckels relativ zum Aufnahmegefäss um mindestens 45° oder um mindestens 90° ermöglicht eine ausreichend grosse bzw. gar komplette Freilegung des vom Aufnahmegefäss begrenzten Teil des Innenraums nach oben hin. Das Aufnahmegerät ist dadurch insbesondere für den Einsatz in Isolatoren mit vertikaler Luftströmung gut geeignet. Eine Verschwenkung um sogar mindestens 180° ermöglicht eine optimale Luftzirkulation zum freigelegten Aufnahmegefäss von allen Seiten her, das heisst das Aufnahmegerät ist dann nicht nur für den Einsatz in Isolatoren mit vertikaler Luftströmung gut geeignet, sondern auch für den Einsatz in Isolatoren mit horizontaler Luftströmung.

Vorzugsweise ist der Winkelbereich, über welchen der Verschlussdeckel relativ zum Aufnahmegefäss um das Drehgelenk schwenkbar ist, wählbar. Das heisst, dass bevorzugt mittels der Fernbedienung eingestellt werden kann, über welchen Winkelbereich der Verschlussdeckel bei Öffnen geschwenkt wird, oder dass das Schwenken des Verschlussdeckels mittels der Fernbedienung z.B. nach einem gewissen Winkelbereich gestoppt werden kann. Das vom Nährmediumträger getragene Nährmedium kann dadurch optimal in der Luftströmung des Isolators positioniert und dieser ausgesetzt werden, wobei es sich beliebig um eine horizontale und/oder vertikale Luftströmung handeln kann. Das Aufnahmegerät weist bevorzugt eine Steuerung auf, welche das Verschwenken des Verschlussdeckels mittels des Antriebs steuert, wobei das Aufnahmegerät ausserdem vorteilhaft ein Speicherelement hat zum Abspeichern des Winkelbereichs, um welchen der Verschlussdeckel beim Öffnen zu Verschwenken ist. Auf diese Weise kann die Öffnungsstellung des Verschlussdeckels relativ zum Aufnahmegefäss an die jeweilige Anwendung und die im Isolator vorliegenden Bedingungen angepasst werden.

Um im verschlossenen Zustand eine besonders gute hermetische Abdichtung des Innenraums nach aussen hin zu erreichen und zu gewährleisten, weist das Aufnahmegerät bevorzugt eine vollständig umlaufende, zumindest doppelt ausgeführte Dichtlippe auf.

Die vorliegende Erfindung betrifft ausserdem ein Verfahren zum Erkennen und insbesondere zur Überwachung von Keimen in der Atmosphäre eines Isolators, wobei das Verfahren zumindest die folgenden Schritte aufweist:
- Einbringen eines Aufnahmegeräts, welches bevorzugt wie oben angegeben ausgebildet ist, in einen Raum des Isolators, wobei das Aufnahmegerät einen hermetisch verschlossenen Innenraum aufweist, in welchem ein Nährmediumträger, wie insbesondere eine Petrischale, aufgenommen ist;
- Hermetisches Verschliessen des Raumes des Isolators mit dem als Ganzes darin aufgenommenen Aufnahmegerät; sowie
- Öffnen des Innenraums des Aufnahmegeräts mittels einer Fernbedienung, welche ausserhalb des hermetisch verschlossenen Raumes des Isolators angeordnet ist.

Die oben erwähnten Verfahrensschritte werden bevorzugt in der angegebenen Reihenfolge durchgeführt. Das Verfahren kann aber noch beliebige weitere Verfahrensschritte aufweisen.

Beim Öffnen des Innenraums ist die Fernbedienung üblicherweise in einem den Isolator umgebenden Arbeitsraum angeordnet. Die Bedienung der Fernbedienung erfolgt in der Regel durch einen Benutzer, also einen Menschen. In gewissen Ausführungsformen kann die Fernbedienung auch eine Schnittstelle zu einer Rechner- oder Steuereinheit aufweisen, um eine maschinengesteuerte Bedienung der Fernbedienung zu ermöglichen.

Im Anschluss an die erwähnten Verfahrensschritte wird der Nährmediumträger mit von diesem getragenen Nährmedium typischerweise eine gewisse Zeit lang, welche als Einwirkungszeit bezeichnet wird, der Atmosphäre des hermetisch verschlossenen Raumes des Isolators ausgesetzt. Allfällig in der Atmosphäre des Isolatorraums vorhandene Keime können sich während dieser Einwirkungszeit auf dem Nährmedium absetzen.

Das Verfahren kann ausserdem die folgenden Schritte aufweisen, welche bevorzugt anschliessend zu den oben genannten Schritten ausgeführt werden:
- Hermetisches Verschliessen des Innenraums des Aufnahmegeräts mittels der Fernbedienung nach einer gewissen Einwirkungszeit, während welcher ein vom Nährmediumträger getragenes Nährmedium der Atmosphäre des hermetisch verschlossenen Raumes des Isolators ausgesetzt war;
- Öffnen des Raumes des Isolators; sowie
- Entnehmen des Aufnahmegeräts als Ganzes aus dem Raum des Isolators.

Zum Öffnen des Raums des Isolators weist der Isolator bevorzugt eine Zugangsöffnung auf, welche insbesondere zum Einbringen und Entnehmen des Aufnahmegeräts dient. Die Zugangsöffnung kann als Schleuse ausgebildet sein, welche ein Einbringen bzw. Entnehmen des Aufnahmegeräts derart erlaubt, dass die Atmosphäre im Isolatorraum dadurch gar nicht oder nur zu einem geringen Masse beeinflusst wird. Das Einbringen und/oder Entnehmen des Aufnahmegeräts erfolgt üblicherweise durch einen Benutzer, zum Beispiel durch Laborpersonal, kann alternativ aber auch durch eine Maschine, wie zum Beispiel einen Roboter erfolgen.

Der Nährmediumträger kann eine Schale und einen Deckel aufweisen. Der Deckel wird dann bevorzugt beim Öffnen des Innenraums des Aufnahmegeräts von der Schale abgehoben. Beim Öffnen des Aufnahmegeräts wird bevorzugt also gleichzeitig das im Nährmediumträger enthaltene Nährmedium zugänglich gemacht. Bevorzugt wird der Deckel beim Verschliessen des Innenraums des Aufnahmegeräts wieder auf die Schale aufgesetzt.

Die Schale und der Deckel des Nährmediumträgers können miteinander verriegelbar sein. In diesem Fall wird der Deckel bevorzugt im hermetisch verschlossenen Innenraum des Aufnahmegeräts, insbesondere bevorzugt noch bevor das Aufnahmegerät in den Raum des Isolators eingebracht wird, gegenüber der Schale entriegelt. Die Entriegelung erfolgt dabei vorzugsweise von Hand mittels eines am Aufnahmegerät dafür vorgesehenen Betätigungselements.

Nach der Einwirkungszeit wird der Deckel bevorzugt im erneut hermetisch verschlossenen Innenraum des Aufnahmegeräts, insbesondere bevorzugt nachdem das Aufnahmegerät aus dem Raum des Isolators entnommen wurde, mit der Schale verriegelt. Die Verriegelung erfolgt dabei vorzugsweise von Hand mittels eines am Aufnahmegerät dafür vorgesehenen Betätigungselements.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht eines Isolators mit einem darin eingebrachten Aufnahmegerät gemäss einer erfindungsgemässen Ausführungsform;
- Fig. 2: eine perspektivische Ansicht eines Aufnahmegeräts gemäss einer erfindungsgemässen Ausführungsform, in geschlossenem Zustand;
- Fig. 3: eine Seitenansicht des Aufnahmegeräts der Fig. 2;
- Fig. 4: eine Ansicht des Aufnahmegeräts der Fig. 2, in vollständig geöffnetem Zustand, mit einem darin gehaltenen Nährmediumträger;
- Fig. 5: eine Ansicht des Aufnahmegeräts der Fig. 2, in vollständig geöffnetem Zustand, ohne Nährmediumträger;
- Fig. 6: eine Ansicht des Aufnahmegeräts der Fig. 2, in teilweise geöffnetem Zustand, mit einem darin gehaltenen Nährmediumträger;
- Fig. 7: eine zentrale Schnittansicht durch das Aufnahmegefäss des Aufnahmegeräts der Fig. 2, mit darin aufgenommenem Nährmediumträger;
- Fig. 8: eine vergrösserte Detailansicht des in Fig. 7 gestrichelt umrandeten Bereichs;
- Fig. 9: eine perspektivische Explosionsansicht des Aufnahmegefässes des Aufnahmegeräts der Fig. 2;
- Fig. 10: eine vergrösserte Detailansicht des in Fig. 9 gestrichelt umrandeten Bereichs;
- Fig. 11: eine zentrale Schnittansicht durch den Verschlussdeckel des Aufnahmegeräts der Fig. 2;
- Fig. 12: eine vergrösserte Detailansicht des in Fig. 11 gestrichelt umrandeten Bereichs;
- Fig. 13: eine zentrale Schnittansicht durch den Verschlussdeckel und das Aufnahmegefäss des Aufnahmegeräts der Fig. 2, in geschlossenem Zustand, mit einem darin aufgenommenen Nährmediumträger;
- Fig. 14: eine perspektivische Explosionsansicht von Teilen des Verschlussdeckels des Aufnahmegeräts der Fig. 2; sowie
- Fig. 15: eine perspektivische Explosionsansicht des im Verschlussdeckel des Aufnahmegeräts der Fig. 2 untergebrachten Verriegelungsmechanismus'.

### BESCHREIBUNG BEVORZUGTER AUSFÜHRUNGSFORMEN

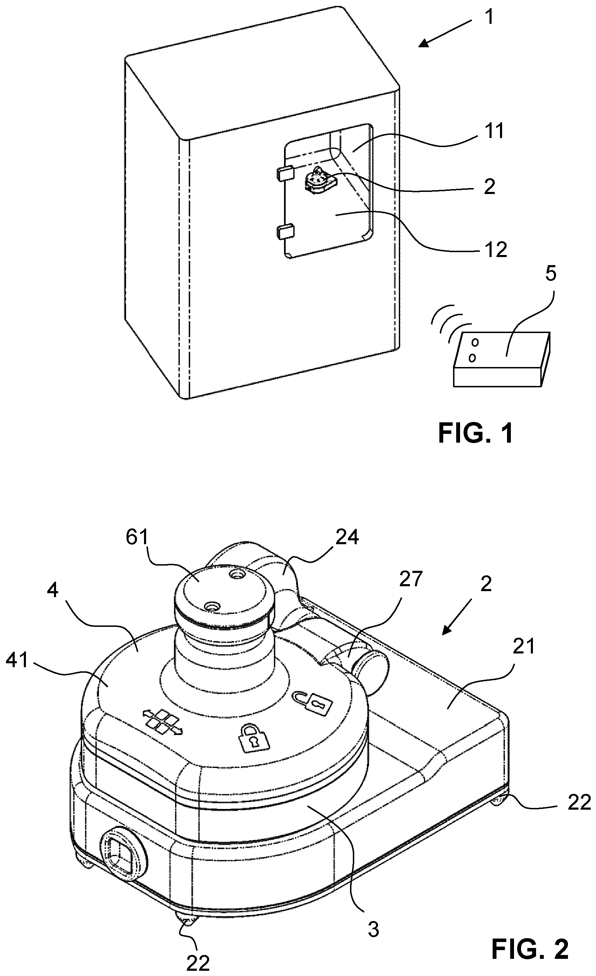

Die Figur 1 zeigt einen Isolator 1 mit einem darin eingebrachten, erfindungsgemässen Aufnahmegerät 2. Die Figuren 2 bis 15 zeigen das erfindungsgemässe Aufnahmegerät 2 sowie Teile davon in unterschiedlichen Ansichten.

Wie in der Figur 1 erkennbar ist, wurde das Aufnahmegerät 2 als Ganzes durch eine Zugangsöffnung 12 des Isolators 1 hindurch in einen hermetisch isolierbaren Raum 11 des Isolators 1 eingebracht. Im Aufnahmegerät 2 ist ein in der Figur 1 nicht sichtbarer Nährmediumträger aufgenommen, der in einem Innenraum des Aufnahmegeräts 2 angeordnet und hermetisch gegenüber der Umgebung, also hier gegenüber des Raumes 11 des Isolators 1, isoliert ist. Der Nährmediumträger, welcher ein Nährmedium trägt, wurde in das vorgängig sterilisierte Aufnahmegerät 2 eingebracht, bevor dieses, nach einer allenfalls erneuten Sterilisation, in den Isolator 1 eingesetzt wurde. Beim Isolator 1 kann es sich insbesondere um handschuhlose Isolatoren wie, aber nicht ausschliesslich, den sog. Produktisolator Vanrx SA25 der Firma Vanrx (zugehörig zu Cytiva), Kanada handeln.

Nachdem die Zugangsöffnung 12 geschlossen und der Raum 11 nach aussen hin hermetisch abgedichtet wurde, kann eine Sterilisation des Raums 11 durchgeführt werden. Hierzu kann insbesondere, aber nicht ausschliesslich, eine sog. VHP(Vaporized Hydrogene Peroxyde)-Dekontamination durchgeführt werden. Der Nährmediumträger ist während dieser Zeit durch das Aufnahmegerät 2 geschützt. Wenn der Isolator für die Produktion bereit ist, kann das Aufnahmegerät 2 im nun geschlossenen und sterilisierten Isolator 1 mittels einer Fernbedienung 5 von aussen her drahtlos geöffnet und das auf dem Nährmediumträger angeordnete Nährmedium dadurch der Atmosphäre im Raum 11 des Isolators 1 ausgesetzt werden. Das Nährmedium kann so während einer gewissen Einwirkungszeit zum Bio-Monitoring verwendet werden, das heisst allfällig im Raum 11 vorhandene Keime setzen sich auf dem Nährmedium ab und können dadurch detektiert werden: Das Nährmedium kann in einem weiteren Arbeitsschritt ausserhalb des Isolators 1 bebrütet werden, um die entstandenen Keimpopulationen anschliessend identifizieren zu können.

Nach Ablauf der Einwirkungszeit wird das Aufnahmegerät 2 mittels der Fernbedienung 5 wieder verschlossen, so dass der Nährmediumträger inkl. des Nährmedium hermetisch gegenüber der Umgebung isoliert ist. Das Öffnen und Schliessen des Aufnahmegeräts 2 und somit die gesamte Handhabung des Nährmediumträgers im Inneren des hermetisch verschlossenen Isolators 1 erfolgt somit ausschliesslich mittels der Fernbedienung 5. Es ist weder ein Handschuheingriff noch ein Roboter notwendig. Ein unbeabsichtigtes Verschleppen von Keimen im Raum 11 des Isolators 1 ist dadurch weitgehend ausgeschlossen. Beim Isolator 1 kann es sich um einen herkömmlichen Isolator mit oder ohne Handschuheingriff handeln.

Nach dem Verschliessen des Aufnahmegeräts 2 kann die Zugangsöffnung 12 dann geöffnet und das immer noch verschlossene Aufnahmegerät 2 als Ganzes dem Isolator 1 entnommen werden. Ausserhalb des Isolators 1 kann das Aufnahmegerät 2 dann geöffnet und der Nährmediumträger aus diesem herausgenommen werden, um zum Beispiel bebrütet und/oder analysiert zu werden. Das Aufnahmegerät 2 kann für weitere Verwendungen mit anderen Nährmediumträgern bzw. mit neuen Nährmedien benutzt werden.

Eine mögliche Ausführungsform eines Aufnahmegeräts 2, wie es in der Figur 1 beispielsweise verwendet werden kann, ist in den Figuren 2 bis 15 in verschiedenen

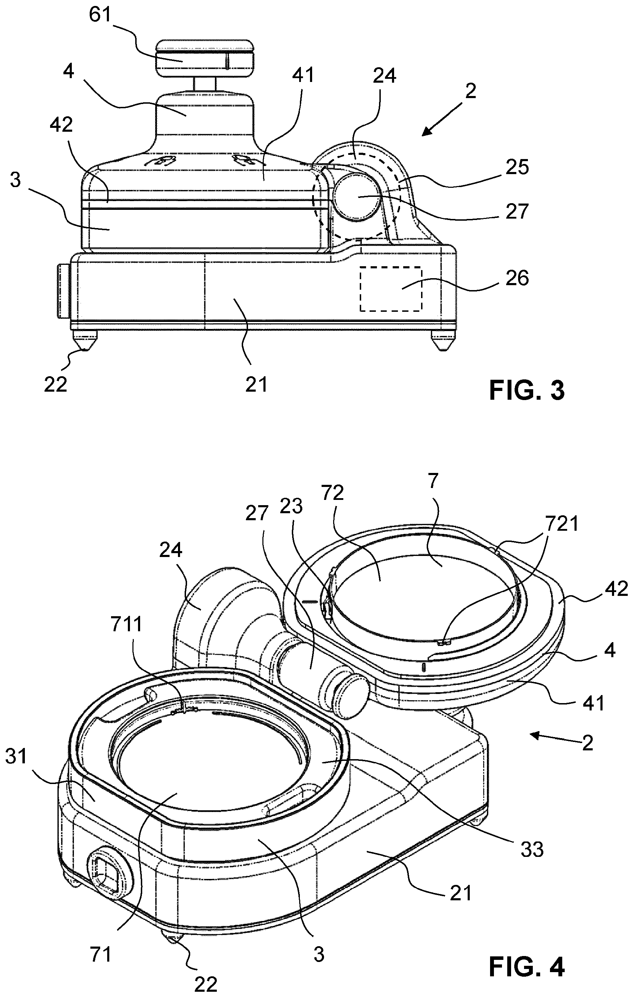

Ansichten gezeigt. Wie in den Figuren 2 und 3 gut erkennbar ist, weist das Aufnahmegerät 2 ein Basisteil 21 auf, auf welchem ein Aufnahmegefäss 3 angeordnet ist, das mittels eines Verschlussdeckels 4 verschliessbar ist. Der Verschlussdeckel 4 ist hierzu relativ zum Aufnahmegefäss 3 um ein Drehgelenk 27 schwenkbar, das seitlich zum Aufnahmegefäss 3 und zum Verschlussdeckel 4 angeordnet ist. Das Drehgelenk 27 verbindet somit das Aufnahmegefäss 3 und den Verschlussdeckel 4 miteinander.

Das Basisteil 21 weist vier Auflagefüsse 22 auf, mit denen das Aufnahmegerät 2 bestimmungsgemäss auf einer ebenen Oberfläche abstellbar ist oder auf bzw. in einer dafür vorgesehenen Halterung platziert werden kann.

Durch das Basisteil 21 wird seitlich zum Drehgelenk 27 ein Antriebsgehäuse 24 gebildet, in welchem ein Antrieb 25 in Form eines Elektromotors untergebracht ist (siehe Figur 3). Der Antrieb 25, welcher mittels der Fernbedienung 5 ansteuerbar ist, dient zum Öffnen und Schliessen des Aufnahmegeräts 2 mittels Verschwenken des Verschlussdeckels 4 um das Drehgelenk 27. Zur Energieversorgung des Antriebs 25 dient eine Batterie 26, welche ebenfalls im Basisteil 21 untergebracht ist, wie es in der Figur 3 angedeutet ist. Die Batterie 26 wird durch ein drahtloses System geladen, z.B. mittels Induktion, so dass am Aufnahmegerät 2 hierfür keine Stecker notwendig sind. Im Basisteil 21 sind ausserdem bevorzugt eine Elektronikeinheit mit Steuerung, ein Speicherelement und/oder eine Drahtlos-Kommunikationseinrichtung angeordnet, welche in den Figuren jedoch alle nicht erkennbar sind.

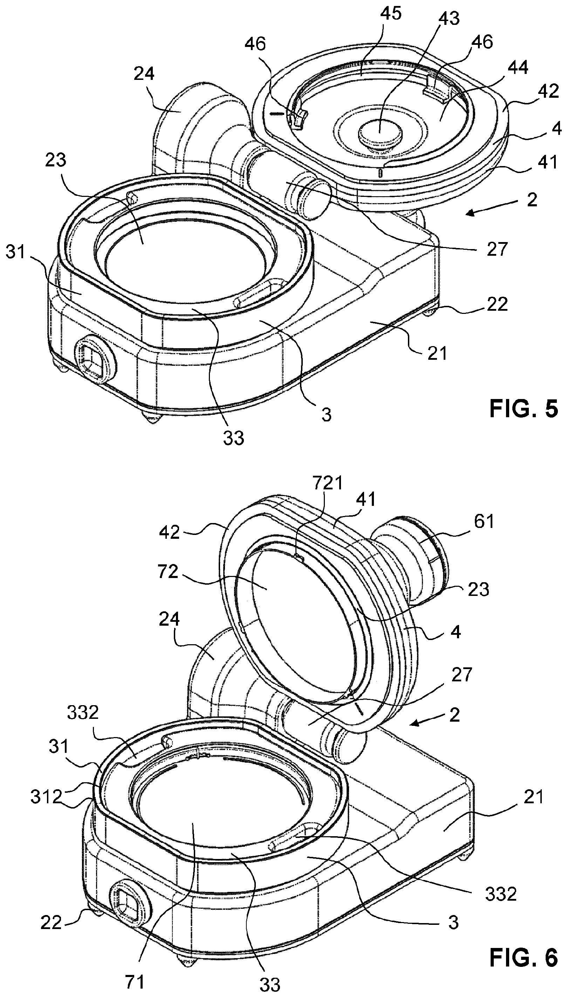

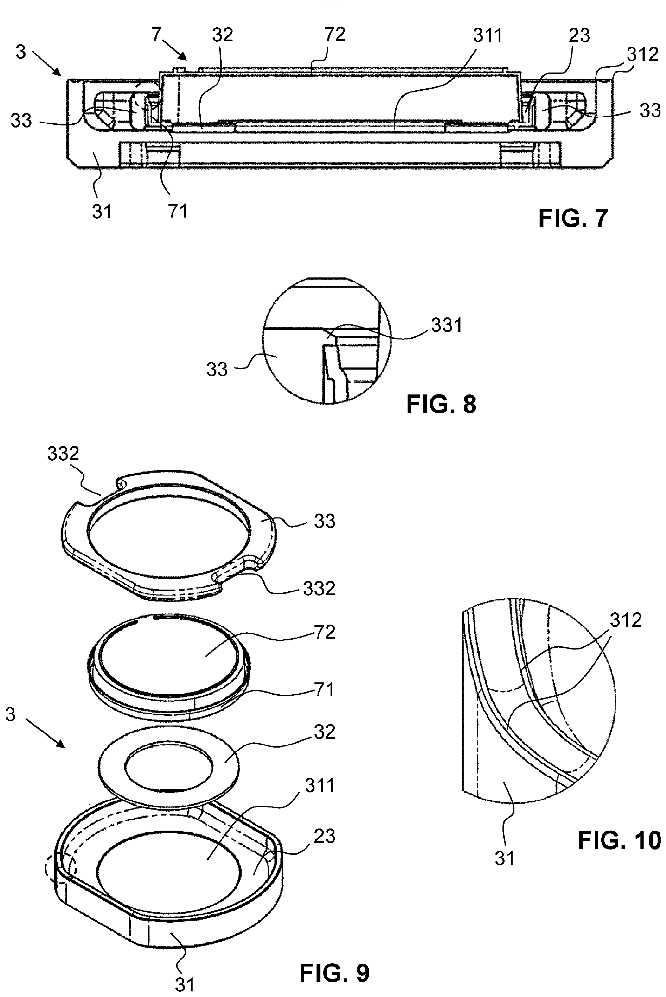

Während das Aufnahmegerät 2 in den Figuren 2 und 3 im verschlossenen Zustand gezeigt ist, zeigen die Figuren 4 und 5 das Aufnahmegerät 2 jeweils im vollständig geöffneten Zustand. Dabei zeigt die Figur 4 das Aufnahmegerät 2 mit und die Figur 5 ohne einem darin eingesetzten Nährmediumträger 7. Der Nährmediumträger 7 ist hier als eine Petrischale ausgebildet mit einer Schale 71 und einem in die Schale 71 einsetzbaren Deckel 72. Schale 71 und Deckel 72 sind mittels Verriegelungselementen 711 und 721 miteinander verriegelbar. Die Verriegelungselemente 711 und 721 bilden dabei bevorzugt, und wie es beim gezeigten Ausführungsbeispiel der Fall ist, gemeinsam einen Bajonettverschluss. Bei vorliegenden Ausführungsbeispiel handelt es sich beim Nährmediumträger 7 um eine unter dem Namen ICRplus Settle Plate bekannte Petrischale der Firma Merck KGaA, Deutschland. Eine Querschnittsansicht durch den geschlossenen und verriegelten Nährmediumträger 7 ist in der Figur 7 gezeigt.

Das Verschwenken des Verschlussdeckels 4 um 180° relativ zum Aufnahmegefäss 3, wie es in den Figuren 4 und 5 gezeigt ist, hat den Vorteil, dass das in der Schale 71 aufgenommene Nährmedium dann optimal der Atmosphäre im Raum 11 des Isolators 1 ausgesetzt werden kann. Die Luft kann dadurch von allen Seiten her zum Nährmedium strömen.

Vorzugsweise ist der Winkelbereich, um welchen der Verschlussdeckel 4 relativ zum Aufnahmegefäss 3 um das Drehgelenk 27 verschwenkbar ist, einstellbar. So kann der Verschlussdeckel 4 beim Öffnen zum Beispiel auch um nur 90° verschwenkt werden, wie es in der Figur 6 gezeigt ist, was je nach Situation und Anwendung vorteilhaft sein kann.

Das Aufnahmegefäss 3 des Aufnahmegeräts 2 ist in den Figuren 7 bis 10 näher gezeigt. Es weist eine Aufnahmeschale 31 mit einer zentralen kreisflächenförmig ausgebildeten Vertiefung 311 auf. In die Vertiefung 311 ist Unterlegring 32 eingelegt, der zur Auflage der Schale 71 des Nährmediumträgers 7 dient. Als Halteelement zum Festhalten der Schale 71 beim Öffnen des Aufnahmegeräts 2 dient ein Fixierring 33, der mit einem sich radial nach innen erstreckenden, umlaufenden Rückhaltesteg 331 zum Aufliegen auf der Schale 71 ausgebildet ist (siehe Figur 7 und insbesondere Figur 8). Durch das Aufliegen des Fixierrings 33 auf der Schale 71 ist diese im geschlossenen Zustand des Aufnahmegeräts 2 nach unten zur Aufnahmeschale 31 hin festgeklemmt und kann sich beim Anheben und/oder Drehen des Verschlussdeckels 4 nicht mit diesem mitbewegen.

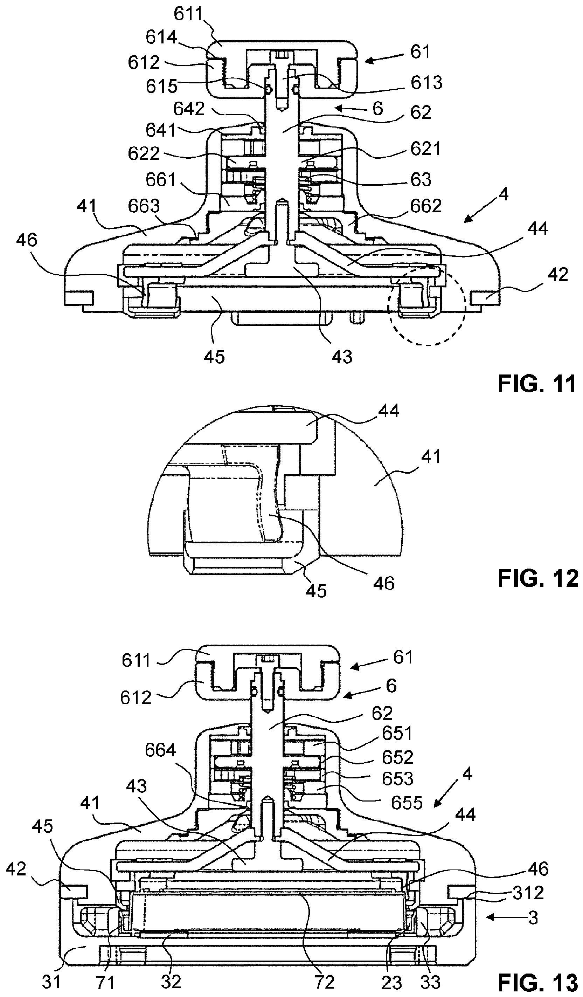

Beim Verschliessen des Aufnahmegeräts 2 kommt ein weiter unten erläuterter Gleitring 45 (siehe Figuren 11 bis 13) zwischen dem Fixierring 33 und dem Verschlussdeckel 4 zu liegen und dient dadurch zum Festklemmen der Schale 71. Ausserdem dient der Fixierring 33 mit seiner zentralen Öffnung nach dem Einsetzen des Nährmediumträgers 7 in das Aufnahmegefäss 3 zum Zentrieren der Schale 71. Am Fixierring 33 sind am äusseren Rand zwei diametral gegenüberliegende Eingriffsaussparungen 332 vorgesehen, welche die Entnahme des Fixierrings 33 aus der Aufnahmeschale 31 erleichtern. Das Entfernen des Fixierrings 33 ist nötig, wenn der Nährmediumträger 7 dem Aufnahmegerät 2 entnommen werden soll.

Der modulare Aufbau des Aufnahmegefässes 3 mit Aufnahmeschale 31, Unterlegring 32 und Fixierring 33 hat neben den bereits erwähnten Vorteilen den zusätzlichen Vorteil, dass das Aufnahmegefäss 3 relativ einfach auch an andere Formen, Grössen und/oder Typen von Nährmediumträgern 7 angepasst werden kann. Es müssen hierzu einfach einer oder mehrere entsprechend angepasste Fixierringe 33 vorgesehen werden.

Beim Verschliessen des Aufnahmegeräts 2 wird ein hermetisch isolierter Innenraum 23 gebildet, welcher gemeinsam und ausschliesslich vom Aufnahmegefäss 3 und vom Verschlussdeckel 4 begrenzt wird. Um eine hermetische Abdichtung zu gewährleisten, weist die Aufnahmeschale zwei vollständig umlaufend ausgebildete Dichtlippen 312 auf (siehe Figur 10), welche jeweils zum umlaufenden Anliegen an einen Dichtring 42 des Verschlussdeckels 4 ausgebildet sind. Der zum Beispiel in den Figuren 6 und 11 erkennbare Dichtring 42 ist in eine im Bereich der Unterseite des Verschlussdeckels 4 angeordnete Nut eingesetzt und erstreckt sich vollständig umlaufend entlang der radialen Aussenseite des Verschlussdeckels 4. Aufgrund der doppelten Ausführung der Dichtlippe 312 wird eine besonders effiziente hermetische Isolierung des Innenraums 23 erreicht und sichergestellt.

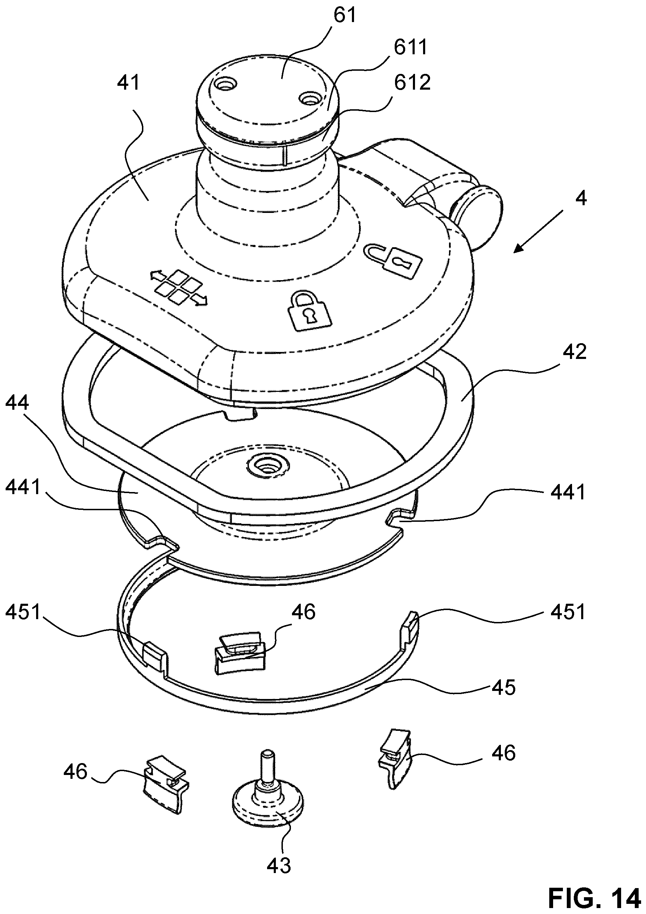

Die Ausbildung des Verschlussdeckels 4 ist in den Figuren 11 bis 15 ersichtlich. Als Grundtragestruktur weist der Verschlussdeckel 4 eine Mantelstruktur 41 auf, wie sie z.B. in den Figuren 11 und 14 gut erkennbar ist. Im unteren Bereich der Mantelstruktur 41 ist an der radialen Aussenseite die bereits erwähnte Nut mit dem darin eingesetzten Dichtring 42 ausgebildet.

Wie zudem in den Figuren 11 und 13 erkennbar ist, weist die Mantelstruktur 41 eine zentrale, vertikal verlaufende Durchgangsöffnung auf, in welcher ein Verriegelungsmechanismus 6 angeordnet ist. An der Unterseite des weiter unten erläuterten Verriegelungsmechanismus' 6 ist eine Halteplatte 44 angebracht, welche das Innenleben des Verschlussdeckels 4 nach unten hin abdeckt und so einen unteren Abschluss des Verschlussdeckels 4 bildet. Die Halteplatte 44 ist im Wesentlichen kreisrund ausgebildet und weist eine zentrale Wölbung nach oben hin auf. Im Zentrum der Wölbung ist eine Durchgangsöffnung ausgebildet, welche zur Fixierung der Hauptplatte 44 an der Betätigungsstange 62 dient. Die Fixierung erfolgt über eine Schraube 43. Die Schraube 43 ist durch die an der Halteplatte 44 vorgesehene Durchgangsöffnung hindurch in eine Betätigungsstange 62 des Verriegelungsmechanismus' 6 eingeschraubt (Figuren 11 und 13), wodurch die Halteplatte 44 drehfest an der Betätigungsstange 62 befestigt ist.

An ihrer Aussenseite weist die Halteplatte 44 radial nach aussen hin offene Aussparungen 441 auf, in welche jeweils ein Klemmteil 46 und ein Rastelement 451 eingesetzt sind. Im fertig montierten Zustand sind das Klemmteil 46 und das Rastelement 451 dadurch jeweils in der von der Halteplatte 44 und der Mantelstruktur 41 begrenzten Aussparung 441 festgehalten. Das Klemmteil 46 ist dabei radial innerhalb des Rastelements 451 angeordnet. Die Rastelemente 451 bilden jeweils ein Teil eines Gleitrings 45, das heisst die Rastelemente 451 sind aufgrund des Gleitrings 45 einstückig miteinander verbunden. Die Rastelemente 451 dienen zur drehfesten Anbringung des Gleitrings 45 an der Halteplatte 44. Durch ihren Einsatz in der Aussparung 441 sind die ansonsten nicht miteinander verbundenen Klemmteile 46 ebenfalls drehfest an der Halteplatte 44 angebracht.

Die sich axial nach unten zum Aufnahmegefäss 3 hin erstreckenden Klemmteile 46 bilden Haltelemente, welche zum radialen Festklemmen des Deckels 72 des Nährmediumträgers 7 ausgebildet sind. Beim Verschliessen des Aufnahmegeräts 2 gleitet zuerst der sich nicht vollständig umlaufend erstreckende Gleitring 45 in axialer Richtung entlang der radialen Aussenseite des Deckels 72 nach unten. Der Deckel 72 wird dadurch bereits entlang eines gewissen, nicht ganz vollständigen Umfangbereiches leicht festgeklemmt. Ausserdem wird der Deckel 72 durch den Gleitring 45 bzgl. des Verschlussdeckels 4 zentriert, so dass die Klemmteile 46 radial an der Aussenseite des Deckels 72 nach unten gleiten und diesen festklemmen können (siehe Figur 13). Der Deckel 72 ist dadurch drehfest an der Halteplatte 44 festgeklemmt.

Die in den Figuren 11, 13 und 14 erkennbare Halteplatte 44 weist eine untere Anschlagfläche auf, die zum Hinunterpressen des Deckels 72 zur Schale 71 hin dient.

Die Funktionsweise des Verriegelungsmechanismus' 6 ergibt sich aus einer Zusammenschau der Figuren 11, 13 und 15: Als zentrales Element weist der Verriegelungsmechanismus 6 die weiter oben bereits erwähnte, sich zentral und in vertikaler Richtung erstreckende Betätigungsstange 62 auf. Am oberen Ende ist mittels einer Schraube 613 ein Betätigungsknauf 61 drehfest an der Betätigungsstange 62 befestigt. Der Betätigungsknauf 61 dient zur manuellen Bedienung des Verriegelungsmechanismus' 6 durch einen Benutzer. Der Betätigungsknauf 61 weist in der vorliegenden Ausführungsform ein oberes Knaufelement 611 und ein unteres Knaufelement 612 auf, welche ineinandergreifend derart zusammengesetzt sind, dass sie gemeinsam einen überwiegenden Grossteil der Aussenfläche des Betätigungsknaufes 61 bilden. Im Bereich ihres radial äusseren Randes ist ein äusserer Dichtring zwischen den beiden Knaufelementen 611 und 612 eingeklemmt, um das Eindringen von Partikeln und Flüssigkeiten in das Innere des Betätigungsknaufes 61 zu verhindern. Ein innerer Dichtring 615 ist in einer radialen Aussennut angeordnet, welche sich an einem oberen Bereich der Betätigungsstange 62 befindet, der sich bis in den Betätigungsknauf 61 hinein erstreckt. Der innere Dichtring 615 ist zwischen Betätigungsstange 62 und dem unteren Knaufelement 612 eingeklemmt und dichtet dadurch diese beiden Elemente gegeneinander ab.

Der Betätigungsknauf 61 kann in anderen Ausführungsformen auch einteilig ausgebildet sein. Je nach Ausführungsform kann der Betätigungskauf 61 zudem von der Betätigungsstange 62 abnehmbar sein, insbesondere ohne Zuhilfenahme eines Werkzeugs abnehmbar sein. Beispielsweise kann der Betätigungsknauf 61 mittels eines Rasteingriffs an der Betätigungsstange 62 anbringbar sein. Durch Abnehmen des Betätigungsknaufs 61 vor dem Einbringen des Aufnahmegeräts 2 in den Isolator 1 kann die Kontaminationswahrscheinlichkeit verringert werden.

Die Betätigungsstange 62 weist einen in der Mitte ihrer Längserstreckung angeordneten, umlaufenden Mittelflansch 621 auf. Auf diametral gegenüberliegenden Seiten erstreckt sich vom Mittelflansch 621 jeweils ein kurzer Führungssteg 622 in radialer Richtung nach aussen hin. Die beiden Führungsstege 622 dienen in Kombination mit einer Führungseinheit 65 des Verriegelungsmechanismus' 6 zum Führen des Deckels 72 beim Entriegeln und Verriegeln des Nährmediumträgers 7 bei geschlossenem Aufnahmegerät 2. Der Mittelflansch 621 und die Führungsstege 622 sind bevorzugt einstückig am vertikalen stangenförmigen Teil der Betätigungsstange 62 angeformt.

Die Führungseinheit 65 weist von oben nach unten betrachtet eine obere Kulissenführung 651, eine mittlere Kulissenführung 652, eine untere Kulissenführung 653 sowie einen Zwischenring 655 auf, welche mittels Verbindungsstiften 654 verdrehsicher miteinander verbunden sind. Die Elemente 651, 652, 653 und 655 sind alle ringförmig ausgebildet mit einer zentralen Durchgangsöffnung, durch welche sich die Betätigungsstange 62 hindurch erstreckt. Bei den Kulissenführungen 651, 652 und 653 ist die zentrale Durchgangsöffnung jedoch jeweils unrund ausgebildet, um eine Kulisse zu bilden, die eine Drehbewegung der Betätigungsstange nur über einen gewissen, vorgegebenen Winkelbereich erlaubt und ein axiales Verschieben der Betätigungsstange nur in gewissen vordefinierten Drehstellungen ermöglicht.

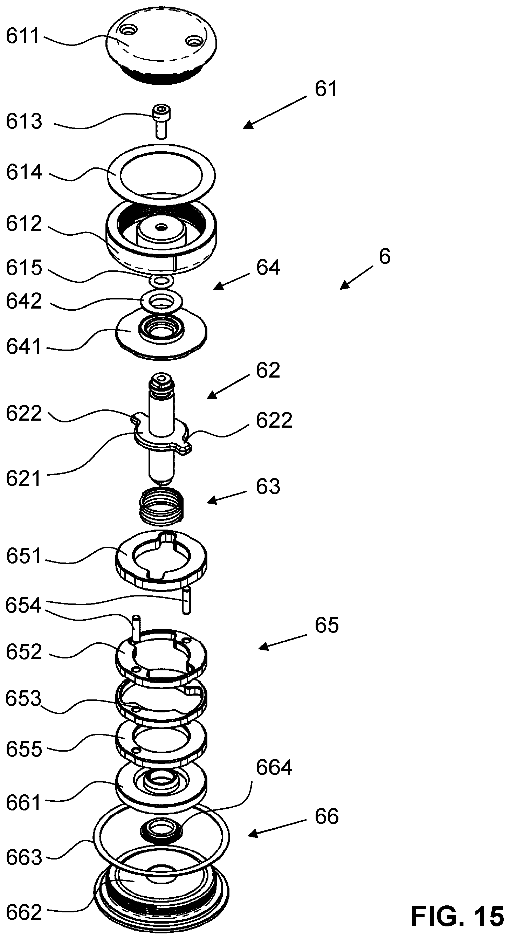

Die Grundstellung der Betätigungsstange 62 und somit des Verriegelungsmechanismus 6 ist in den Figuren 11, 13 und 15 gezeigt: Die Führungsstege 622 der Bestätigungsstange 62 befinden sich auf der Höhe der mittleren Kulissenführung 652. Von oben betrachtet (Figur 15) ist die Betätigungsstange 62 dabei soweit im Gegenuhrzeigersinn gedreht, dass die Führungsstege 622 an der Kulissenführung 652 anliegen. In dieser Grundstellung des Verriegelungsmechanismus' wird der verriegelte Nährmediumträger 7 üblicherweise vom Benutzer in das geöffnete Aufnahmegerät 2 eingesetzt. Die Situation nach dem Verschliessen des Aufnahmegeräts 2 mittels des Antriebs 25 ist in Figur 13 dargestellt. Der Nährmediumträger 7 ist weiterhin verriegelt und im nun hermetisch isolierten Innenraum 23 des Aufnahmegeräts 2 angeordnet. Aufgrund des Drucks, welcher der Verschlussdeckel 4 auf den Fixierring 33 ausübt, wird die Schale 71 durch den Fixierring 33 in der Aufnahmeschale 31 festgeklemmt. Der Deckel 72 wird vom Gleitring 45 geringfügig geklemmt.

Um bei verschlossenem Aufnahmegerät 2 den Nährmediumträger 7 zu entriegeln, das heisst dessen Bajonettverschluss zwischen Schale 71 und Deckel 72 zu betätigen, wird der Betätigungsknauf 61 nun von einem Benutzer manuell nach unten gedrückt und anschliessend in Gegenuhrzeigerrichtung gedreht: Die Betätigungsstange 62 mit ihren Führungsstegen 622 wird dadurch in axialer Richtung auf die Höhe der unteren Kulissenführung 653 vorverschoben. Dabei gerät die Halteplatte 44 in Anlage mit dem Deckel 72 und presst diesen zur Schale 71 hin. Gleichzeitig gleiten die Klemmteile 46 an der Aussenseite des Deckels 72 entlang nach unten und klemmen diesen mit einer erheblich grösseren Klemmkraft fest, als sie vom Gleitring 45 bisher ausgeübt wurde. Der Deckel 72 ist dadurch drehfest an der Halteplatte 44 und somit an der Betätigungsstange 62 gehalten. Durch die anschliessende erwähnte Drehung der Betätigungsstange 62 in Gegenuhrzeigerrichtung wird der Deckel 72 gegenüber der Schale 71 gedreht, wodurch die Verriegelungselemente 711 und 721 des Bajonettverschlusses ausser Eingriff geraten. Der Nährmediumträger 7 ist somit entriegelt und das Aufnahmegerät 2 kann geöffnet werden. Aufgrund der Festklemmung des Deckels 72 durch die Klemmteile 46 wird dieser beim Öffnen mit dem Verschlussdeckel 4 mitbewegt und von der Schale 71 abgehoben. Das im Nährmediumträger 7 enthaltene Nährmedium ist somit der umgebenden Atmosphäre ausgesetzt.

Nach dem Wiederverschliessen des Aufnahmegeräts 2 mittels des Antriebs 25 ist der Innenraum 23 des Aufnahmegeräts 2 mit dem darin aufgenommenen Nährmediumträger 7 wieder hermetisch von der Umgebung isoliert. Der Nährmediumträger 7 ist aber immer noch entriegelt, das heisst die Verriegelungselemente 711 und 721 des Bajonettverschlusses greifen nicht ineinander. Der Verriegelungsmechanismus 6 befindet sich immer noch in derselben Stellung wie beim Öffnen des Aufnahmegeräts 2, das heisst die Führungsstege 622 der Betätigungsstange 62 sind auf der Höhe der unteren Kulissenführung 653 angeordnet.

Um bei verschlossenem Aufnahmegerät 2 den Nährmediumträger 7 zu verriegeln, das heisst Schale 71 und Deckel 72 mittels des Bajonettverschlusses miteinander zu verriegeln, wird der Betätigungsknauf 61 vom Benutzer manuell in Uhrzeigerrichtung gedreht und anschliessend nach oben gezogen: Durch das Drehen in Uhrzeigerrichtung wird der weiterhin von den Klemmteilen 46 festgehaltene Deckel 72 mitgedreht, wodurch die Verriegelungselemente 711 und 721 der Schale 71 und des Deckels 72 wieder ein Eingriff miteinander geraten und den Nährmediumträger 7 dadurch verriegeln. Beim anschliessenden axialen Zurückziehen des Betätigungsknaufs 61 nach oben hin werden die Klemmteile 46 gegenüber dem Deckel 72 zurückgezogen, da dieser nun aufgrund des verriegelten Bajonettverschlusses an der Schale 71 gehalten ist. Der Deckel 72 wird somit nicht mehr von den Klemmteilen 46 festgeklemmt, und der Verriegelungsmechanismus 6 befindet sich wieder in seiner in den Figuren 11, 13 und 15 gezeigten Grundposition.

Das Aufnahmegerät 2 kann dann mittels des Antriebs 25 wieder geöffnet werden, wobei beim Öffnen der Deckel 72 auf der Schale 71 verbleibt. Dies, weil der Deckel 72 erstens via den Bajonettverschluss an der Schale 71 gehalten ist und er zweitens von den Klemmteilen 46 nicht mehr festgeklemmt wird.

Eine weitere Stellung des Verriegelungsmechanismus' 6, welche nachfolgend als Demontageposition bezeichnet wird, wird durch die obere Kulissenführung 651 ermöglicht. Hierzu muss der Betätigungsknauf 61 vom Benutzer aus der Grundposition in Uhrzeigerrichtung gedreht und anschliessend nach oben gezogen werden: Da die obere Kulissenführung 651 eine radiale Innenfläche hat, die gerade den Mittelflansch 621 sowie die Führungsstege 622 komplementär abbildet, ist in der Demontageposition jede weitere Drehung der Betätigungsstange 62 verunmöglicht. In der Demontageposition ist die Betätigungsstange 62 mitsamt der daran angebrachten Halteplatte 44 somit gegenüber der in den Figuren 11 und 13 gezeigten Situation weiter nach oben in die Mantelstruktur 41 hinein zurückgezogen. Die Demontageposition dient der erleichterten Demontage des Verschlussdeckels 4 beispielsweise zu Reinigungs- und/oder Sterilisationszwecken. Mittels Herunterdrücken des Betätigungsknaufs 61 aus der Demontageposition und anschliessender Drehung im Gegenuhrzeigersinn kann der Verriegelungsmechanismus 6 einfach wieder in die Grundposition gebracht werden.

In Bezug auf den Verriegelungsmechanismus 6 sind bevorzugt auf der Aussenseite des Verschlussdeckels 4 und des Betätigungsknaufes 61 Markierungen angebracht, welche es einem Benutzer ermöglichen, die Demontageposition, die Grundposition sowie die Position zu erkennen, bei welcher der Nährmediumträger 7 im Inneren des Aufnahmegeräts 2 entriegelt ist. Entsprechende Markierungen sind in den Figuren 2 und 14 erkennbar.

Wie aus den Figuren 11, 13 und 15 ausserdem erkennbar ist, kann der Verriegelungsmechanismus 6 eine Druckfeder 63 aufweisen, um die Betätigungsstange 62 axial in Richtung der Grundposition bzw. Demontageposition mit einer Kraft zu beaufschlagen. Die Druckfeder 63 kann insbesondere als Spiralfeder ausgebildet sein, welche sich um die Betätigungsstange 62 herum erstreckt und in axialer Richtung einerseits am Mittelflansch 621 der Betätigungsstange 62 und andererseits an einer relativ zur Mantelstruktur 41 ortsfesten Federanschlagscheibe 661 anliegt.

Die Federanschlagscheibe 661 ist Teil einer unteren Befestigungseinheit 66, zu welcher ausserdem eine untere Befestigungsscheibe 662 gehört. Die untere Befestigungsscheibe 662 ist mit einem Aussengewinde in ein Innengewinde der Mantelstruktur 41 eingeschraubt und bildet dadurch einen unteren Abschluss des Verriegelungsmechanismus' 6. Zur Abdichtung des Verriegelungsmechanismus' 6 und somit des Verschlussdeckels 4 nach unten hin ist ein unterer äusserer Dichtring 663 vorgesehen, welcher umlaufend zwischen der unteren Befestigungsscheibe 662 und der Mantelstruktur 41 angeordnet ist und diese gegeneinander abdichtet. Ein unterer innerer Dichtring 664 dichtet die untere Befestigungsscheibe 662 im Bereich ihrer zentralen Durchgangsöffnung umlaufend gegenüber der Betätigungsstange 62 ab. Die Federanschlagscheibe 661, welche ebenfalls eine zentrale Durchgangsöffnung zum Durchführen der Betätigungsstange 62 aufweist, liegt auf der Oberseite der unten Befestigungsscheibe 662 auf.

Nach oben hin weist der Verriegelungsmechanismus 6 eine obere Befestigungseinheit 64 mit einer oberen Befestigungsscheibe 641 auf. Die obere Befestigungsscheibe 641 ist oberhalb der oberen Kulissenführung 651 angeordnet. Zwischen Mantelstruktur 41 und oberer Befestigungsscheibe 641 ist ein oberer Dichtring 642 eingeklemmt, um die Mantelstruktur 41 und die obere Befestigungsscheibe 641 gegenüber der Betätigungsstange 62 abzudichten. Die Betätigungsstange 62 erstreckt sich durch eine zentrale Durchgangsöffnung der oberen Befestigungsscheibe 641 hindurch.

Die vorliegende Erfindung ist nicht auf die in den Figuren und der Beschreibung dargestellten Ausführungsformen beschränkt und eine Vielzahl von Modifikationen ist möglich. So muss der Nährmediumträger zum Beispiel nicht zwingend einen mit einer Schale verriegelbaren Deckel aufweisen. Der Nährmediumträger könnte genausogut durch eine ebene Platte gebildet sein. Dementsprechend muss das Aufnahmegerät, auch wenn dies bevorzugt ist, nicht zwingend einen Verriegelungsmechanismus aufweisen. Das Öffnen des Aufnahmegerät muss zudem nicht unbedingt mittels Verschwenken eines Verschlussdeckels erfolgen. Der Verschlussdeckel könnte in bestimmten Ausführungsformen zum Öffnen zum Beispiel auch translatorisch vom Aufnahmegefäss wegbewegt oder gegenüber einer Fensteröffnung verschoben werden. Falls ein Verriegelungsmechanismus vorhanden ist, kann dieser anstatt manuell auch mittels eines motorischen Antriebs bedienbar sein. Verschiedene der beschriebenen Einzelelemente sind zudem nicht zwingend notwendig und könnten bei anderen Ausführungsformen durchaus auch entfallen oder anders ausgebildet sein. Die Führungskulissen 651, 653 und 654 sowie allenfalls die obere Befestigungsscheibe 641, die Federanschlagscheibe 661 und/oder die untere Befestigungsscheibe 662 könnten beispielsweise gemeinsam durch ein einstückiges Element gebildet sein, wodurch die Verbindungsstifte 654 entfallen könnten. Weitere Änderungen sind möglich.

**BEZUGSZEICHENLISTE**

| | | | |
|---|---|---|---|
| 1 | Isolator | 31 | Aufnahmeschale |
| 11 | Hermetisch isolierbarer | 311 | Vertiefung |
| | Raum | 312 | Dichtlippe |
| 12 | Zugangsöffnung | 32 | Unterlegring |
| | | 33 | Fixierring |
| 2 | Aufnahmegerät | 331 | Rückhaltesteg |
| 21 | Basisteil | 332 | Eingriffsaussparung |
| 22 | Auflagefuss | | |
| 23 | Innenraum | 4 | Verschlussdeckel |
| 24 | Antriebsgehäuse | 41 | Mantelstruktur |
| 25 | Antrieb | 42 | Dichtring |
| 26 | Batterie | 43 | Schraube |
| 27 | Drehgelenk | 44 | Halteplatte |
| | | 441 | Aussparung |
| 3 | Aufnahmegefäss | 45 | Gleitring |
| 451 | Rastelement | 641 | Obere Befestigungsscheibe |
| 46 | Klemmteil | 642 | Oberer Dichtring |
| 5 | Fernbedienung | 65 | Führungseinheit |
| | | 651 | Obere Kulissenführung |
| 6 | Verriegelungsmechanismus | 652 | Mittlere Kulissenführung |
| | | 653 | Untere Kulissenführung |
| 61 | Betätigungsknauf | 654 | Verbindungsstift |
| 611 | Oberes Knaufelement | 655 | Zwischenring |
| 612 | Unteres Knaufelement | | |
| 613 | Schraube | 66 | Untere Befestigungseinheit |
| 614 | Äusserer Dichtring | 661 | Federanschlagsscheibe |
| 615 | Innerer Dichtring | 662 | Untere Befestigungsscheibe |
| | | 663 | Unterer äusserer Dichtring |
| 62 | Betätigungsstange | 664 | Unterer innerer Dichtring |
| 621 | Mittelflansch | | |
| 622 | Führungssteg | 7 | Nährmediumträger |
| | | 71 | Schale |
| 63 | Druckfeder | 711 | Verriegelungselement |
| | | 72 | Deckel |
| 64 | Obere Befestigungseinheit | 721 | Verriegelungselement |

## Patentansprüche

1. Aufnahmegerät (2) für einen Nährmediumträger (7), wie insbesondere eine Petrischale, aufweisend
einen hermetisch verschliessbaren Innenraum (23) zur Aufnahme des Nährmediumträgers (7); sowie
einen drahtlos fernbedienbaren Antrieb (25) zum Öffnen und Verschliessen des Aufnahmegeräts (2), um den Zugang zum Nährmediumträger (7) freizugeben bzw. den Innenraum (23) mit dem darin aufgenommenen Nährmediumträger (7) hermetisch zu verschliessen;
**dadurch gekennzeichnet, dass**
das Aufnahmegerät (2) als Ganzes in einen hermetisch isolierbaren Raum (11) eines Isolators (1) eingebracht und diesem entnommen werden kann.

2. Aufnahmegerät (2) nach Anspruch 1, ausserdem aufweisend zumindest ein Energiespeicherelement, wie insbesondere eine Batterie (26), zur Versorgung des Antriebs (25) mit elektrischer Energie.

3. Aufnahmegerät (2) nach Anspruch 1 oder 2, wobei das Aufnahmegerät (2) dazu ausgebildet ist, beim Öffnen einen Deckel (72) des Nährmediumträgers (7) von einer Schale (71) des Nährmediumträgers (7) abzuheben und beim Verschliessen den Deckel (72) wieder auf die Schale (71) aufzusetzen.

4. Aufnahmegerät (2) nach Anspruch 3, aufweisend ein erstes Halteelement (44, 45, 46) zum Festhalten des Deckels (72) sowie ein zweites Halteelement (33) zum Festhalten der Schale (71) beim Öffnen und Verschliessen des Aufnahmegeräts (2).

5. Aufnahmegerät (2) nach Anspruch 3 oder 4, wobei der Deckel (72) mit der Schale (71) verriegelbar ist, und wobei das Aufnahmegerät (2) dazu ausgebildet ist, den im hermetisch verschlossenen Innenraum (23) aufgenommenen Nährmediumträger (7) zu entriegeln und zu verriegeln.

6. Aufnahmegerät (2) nach Anspruch 5, aufweisend einen Verriegelungsmechanismus (6) zur Entriegelung und Verriegelung eines Nährmediumträgers (7), welcher einen Gewinde- oder Bajonettverschluss (711, 721) aufweist.

7. Aufnahmegerät (2) nach Anspruch 6, wobei der Verriegelungsmechanismus (6) ein erstes Halteelement (44, 45, 46) aufweist zum Festhalten des Deckels (72), und wobei das erste Halteelement (44, 45, 46) aus einer Ausgangsstellung in eine niedergedrückte Stellung und aus der niedergedrückten Stellung in eine niedergedrückte gedrehte Stellung bewegbar ist, um einen Nährmediumträger (7), welcher einen Bajonettverschluss (711, 721) aufweist, zu verriegeln.

8. Aufnahmegerät (2) nach einem der vorhergehenden Ansprüche, aufweisend ein Aufnahmegefäss (3) und einen Verschlussdeckel (4), welche gemeinsam den Innenraum (23) begrenzen, wobei der Verschlussdeckel (4) zum Öffnen und Verschliessen des Innenraums (23) über einen Winkelbereich von mindestens 45°, bevorzugt von mindestens 90°, insbesondere bevorzugt von mindestens 180°, um ein Drehgelenk (27) schwenkbar ist.

9. Aufnahmegerät (2) nach Anspruch 8, wobei der Winkelbereich, über welchen der Verschlussdeckel (4) um das Drehgelenk (27) schwenkbar ist, wählbar ist.

10. Aufnahmegerät (2) nach einem der vorhergehenden Ansprüche, aufweisend eine vollständig umlaufende, zumindest doppelt ausgeführte Dichtlippe (312), um den Innenraum (23) im verschlossenen Zustand hermetisch abzudichten.

11. Verfahren zum Erkennen von Keimen in der Atmosphäre eines Isolators (1), aufweisend zumindest die folgenden Schritte:
- Einbringen eines bevorzugt nach einem der vorhergehenden Ansprüche ausgebildeten Aufnahmegeräts (2) in einen Raum (11) des Isolators (1), wobei das Aufnahmegerät (2) einen hermetisch verschlossenen Innenraum (23) aufweist, in welchem ein Nährmediumträger (7), wie insbesondere eine Petrischale, aufgenommen ist;
- Hermetisches Verschliessen des Raumes (11) des Isolators (1) mit dem als Ganzes darin aufgenommenen Aufnahmegerät (2); sowie
- Öffnen des Innenraums (23) des Aufnahmegeräts (2) mittels einer Fernbedienung (5), welche ausserhalb des hermetisch verschlossenen Raumes (11) des Isolators (1) angeordnet ist.

12. Verfahren nach Anspruch 11, ausserdem aufweisend die folgenden Schritte:
- Hermetisches Verschliessen des Innenraums (23) des Aufnahmegeräts (2) mittels der Fernbedienung (5) nach einer gewissen Einwirkungszeit, während welcher ein vom Nährmediumträger (7) getragenes Nährmedium der Atmosphäre des hermetisch verschlossenen Raumes (11) des Isolators (1) ausgesetzt war;
- Öffnen des Raumes des Isolators (11); sowie
- Entnehmen des Aufnahmegeräts (2) als Ganzes aus dem Raum (11) des Isolators (1).

13. Verfahren nach Anspruch 11 oder 12, wobei der Nährmediumträger (7) eine Schale (71) und einen Deckel (72) aufweist, und wobei der Deckel (72) beim Öffnen des Innenraums (23) des Aufnahmegeräts (2) von der Schale (71) abgehoben wird.

14. Verfahren nach Anspruch 13, wobei die Schale (71) und der Deckel (72) des Nährmediumträgers (7) miteinander verriegelbar sind, und wobei der Deckel (72) im hermetisch verschlossenen Innenraum (23) des Aufnahmegeräts (2), bevorzugt noch bevor das Aufnahmegerät (2) in den Raum (11) des Isolators (7) eingebracht wird, gegenüber der Schale (71) entriegelt wird.

## Claims

1. A receiving device (2) for a culture medium carrier (7), such as in particular a Petri dish, comprising
a hermetically sealable interior space (23) for receiving the culture medium carrier (7); and
a wirelessly remote-controllable drive (25) for opening and closing the receiving device (2) in order to allow access to the culture medium carrier (7) or to hermetically seal the interior space (23) with the culture medium carrier (7) received therein;
**characterized in that**
the receiving device (2) as a whole can be inserted into and removed from a hermetically isolatable space (11) of an isolator (1).

2. The receiving device (2) according to claim 1, additionally comprising at least one energy storage element, such as in particular a battery (26), for supplying the drive (25) with electrical energy.

3. The receiving device (2) according to claim 1 or 2, wherein the receiving device (2) is adapted to lift a lid (72) of the culture medium carrier (7) from a dish (71) of the culture medium carrier (7) when opened and to put the lid (72) back onto the dish (71) when closed.

4. The receiving device (2) according to claim 3, comprising a first retaining element (44, 45, 46) for retaining the lid (72) and a second retaining element (33) for retaining the dish (71) when the receiving device (2) is opened and closed.

5. The receiving device (2) according to claim 3 or 4, wherein the lid (72) can be locked to the dish (71), and wherein the receiving device (2) is adapted to unlock and lock the culture medium carrier (7) received in the hermetically sealed interior space (23).

6. The receiving device (2) according to claim 5, comprising a locking mechanism (6) for unlocking and locking a culture medium carrier (7) which comprises a threaded or bayonet lock (711, 721).

7. The receiving device (2) according to claim 6, wherein the locking mechanism (6) comprises a first retaining element (44, 45, 46) for retaining the lid (72), and wherein the first retaining element (44, 45, 46) is movable from an initial position to a depressed position and from the depressed position to a depressed rotated position in order to lock a culture medium carrier (7) having a bayonet lock (711, 721).

8. The receiving device (2) according to one of the preceding claims, comprising a receiving vessel (3) and a closure lid (4), which together delimit the interior space (23), wherein the closure lid (4) can be pivoted about a rotary joint (27) over an angular range of at least 45°, preferably at least 90°, and in particular preferably at least 180°, in order to open and close the interior space (23).

9. The receiving device (2) according to claim 8, wherein the angular range over which the closure lid (4) can be pivoted about the pivot joint (27) is selectable.

10. he receiving device (2) according to one of the preceding claims, comprising a completely circumferential, at least double sealing lip (312) for hermetically sealing the interior space (23) in the closed state.

11. A method for detecting germs in the atmosphere of an isolator (1), comprising at least the following steps:
- Inserting a receiving device (2), preferably a receiving device (2) according to one of the preceding claims, into a space (11) of the isolator (1), wherein the receiving device (2) comprises a hermetically sealed interior space (23) in which a culture medium carrier (7), such as in particular a Petri dish, is received;
- Hermetically sealing the space (11) of the isolator (1) with the receiving device (2) received therein as a whole; and
- Opening the interior space (23) of the receiving device (2) by means of a remote control (5) which is located outside the hermetically sealed space (11) of the isolator (1).

12. The method according to claim 11, additionally comprising the following steps:
- Hermetically sealing the interior space (23) of the receiving device (2) by means of the remote control (5) after a certain exposure time during which a culture medium carried by the culture medium carrier (7) was exposed to the atmosphere of the hermetically sealed space (11) of the isolator (1);
- Opening the space of the isolator (11); and
- Removing the receiving device (2) as a whole from the space (11) of the isolator (1).

13. The method according to claim 11 or 12, wherein the culture medium carrier (7) comprises a dish (71) and a lid (72), and wherein the lid (72) is lifted from the dish (71) when the interior space (23) of the receiving device (2) is opened.

14. The method according to claim 13, wherein the dish (71) and the lid (72) of the culture medium carrier (7) can be locked together, and wherein the lid (72) is unlocked relative to the dish (71) in the hermetically sealed interior space (23) of the receiving device (2), preferably before the receiving device (2) is introduced into the space (11) of the isolator (7).

## Revendications

1. Un dispositif de réception (2) pour un support de milieu nutritif (7), notamment une boîte de Petri, comprenant
un espace intérieur hermétiquement scellable (23) destiné à recevoir le support de milieu nutritif (7) ;
ainsi qu'un actionneur télécommandable sans fil (25) pour ouvrir et fermer le dispositif de réception (2) afin de permettre l'accès au support de milieu nutritif (7) ou de sceller hermétiquement l'espace intérieur (23) avec le support de milieu nutritif (7) y reçu ;
**caractérisé en ce que** le dispositif de réception (2) dans son ensemble peut être inséré dans, et retiré de, un espace pouvant être hermétiquement isolé (11) d'un isolateur (1).

2. Le dispositif de réception (2) selon la revendication 1, comprenant en outre au moins un élément de stockage d'énergie, notamment une batterie (26), pour alimenter l'actionneur (25) en énergie électrique.

3. Le dispositif de réception (2) selon la revendication 1 ou 2, dans lequel le dispositif de réception (2) est configuré pour soulever un couvercle (72) du support de milieu nutritif (7) d'un plateau (71) du support de milieu nutritif (7) lors de l'ouverture et pour remettre le couvercle (72) sur le plateau (71) lors de la fermeture.

4. Le dispositif de réception (2) selon la revendication 3, comprenant un premier élément de retenue (44, 45, 46) pour maintenir le couvercle (72), ainsi qu'un deuxième élément de retenue (33) pour maintenir le plateau (71) lors de l'ouverture et de la fermeture du dispositif de réception (2).

5. Le dispositif de réception (2) selon la revendication 3 ou 4, dans lequel le couvercle (72) peut être verrouillé sur le plateau (71), et dans lequel le dispositif de réception (2) est conçu pour déverrouiller et verrouiller le support de milieu nutritif (7) reçu dans l'espace intérieur hermétiquement scellé (23).

6. Le dispositif de réception (2) selon la revendication 5, comprenant un mécanisme de verrouillage (6) pour déverrouiller et verrouiller un support de milieu nutritif (7) qui possède une fermeture filetée ou à baïonnette (711, 721).

7. Le dispositif de réception (2) selon la revendication 6, dans lequel le mécanisme de verrouillage (6) comporte un premier élément de retenue (44, 45, 46) pour maintenir le couvercle (72), et dans lequel le premier élément de retenue (44, 45, 46) est déplaçable d'une position de départ à une position enfoncée et déplaçable de la position enfoncée à une position enfoncée tournée afin de verrouiller un support de milieu nutritif (7) comportant une fermeture à baïonnette (711, 721).

8. Le dispositif de réception (2) selon l'une des revendications précédentes, comprenant un vaisseau de réception (3) et un couvercle de fermeture (4), qui définissent ensemble l'espace intérieur (23), dans lequel le couvercle de fermeture (4) peut être pivoté autour d'une articulation pivotante (27) afin d'ouvrir et de fermer l'espace intérieur (23), sur une plage angulaire d'au moins 45°, de préférence d'au moins 90°, et particulièrement de préférence d'au moins 180°.

9. Le dispositif de réception (2) selon la revendication 8, dans lequel la plage angulaire sur laquelle le couvercle de fermeture (4) peut pivoter autour de l'articulation pivotante (27) est sélectionnable.

10. Le dispositif de réception (2) selon l'une des revendications précédentes, comprenant une lèvre (312) d'étanchéité entièrement circonférentielle et réalisée au moins en double, pour sceller hermétiquement l'espace intérieur (23) à l'état fermé.

11. Un procédé de détection de germes dans l'atmosphère d'un isolateur (1), comprenant au moins les étapes suivantes :
- insertion d'un dispositif de réception (2), de préférence conçu selon l'une des revendications précédentes, dans un espace (11) de l'isolateur (1), dans lequel le dispositif de réception (2) comporte un espace intérieur hermétiquement scellé (23) dans lequel est reçu un support de milieu nutritif (7), notamment une boîte de Petri ;
- fermeture hermétique de l'espace (11) de l'isolateur (1) avec le dispositif de réception (2) y reçu dans son ensemble; ainsi que
- ouverture de l'espace intérieur (23) du dispositif de réception (2) au moyen d'une télécommande (5) située à l'extérieur de l'espace hermétiquement scellé (11) de l'isolateur (1).

12. Le procédé selon la revendication 11, comprenant en outre les étapes suivantes :
- scellage hermétique de l'espace intérieur (23) du dispositif de réception (2) au moyen de la télécommande (5) après un certain temps d'exposition, pendant lequel un milieu nutritif transporté par le support de milieu nutritif (7) ait été exposé à l'atmosphère de l'espace hermétiquement scellé (11) de l'isolateur (1) ;
- ouverture de la chambre isolante (11) ; ainsi que
- retrait de l'appareil de réception (2) dans son ensemble de l'espace (11) de l'isolateur (1).

13. Le procédé selon la revendication 11 ou 12, dans lequel le support de milieu nutritif (7) comporte un plateau (71) et un couvercle (72), et dans lequel le couvercle (72) est soulevé du plateau (71) lorsque l'intérieur (23) du dispositif de réception (2) est ouvert.

14. Le procédé selon la revendication 13, dans lequel le plateau (71) et le couvercle (72) du support de milieu nutritif (7) sont verrouillables l'un à l'autre, et dans lequel le couvercle (72) est déverrouillé par rapport au plateau (71) dans l'intérieur hermétiquement scellé (23) du dispositif de réception (2), de préférence avant que le dispositif de réception (2) ne soit introduit dans l'espace (11) de l'isolateur (7).
